Europäisches Patentamt

European Patent Office

Office européen des brevets

(19)

(11) **EP 1 318 199 A1**

(12) **EUROPEAN PATENT APPLICATION**

(43) Date of publication:
**11.06.2003 Bulletin 2003/24**

(51) Int Cl.⁷: **C12P 13/22**, C12M 1/36,
C12Q 3/00

(21) Application number: **01204720.5**

(22) Date of filing: **05.12.2001**

| | |
|---|---|
| (84) Designated Contracting States:<br>**AT BE CH CY DE DK ES FI FR GB GR IE IT LI LU<br>MC NL PT SE TR**<br>Designated Extension States:<br>**AL LT LV MK RO SI**<br><br>(71) Applicants:<br>• **DSM N.V.<br>6411 TE Heerlen (NL)**<br>• **DSM Biotech GmbH<br>52428 Jülich (DE)**<br>• **Forschungszentrum Jülich GmbH<br>52425 Jülich (DE)** | (72) Inventors:<br>• **Gerigk, Marc Robert<br>42115 Wuppertal (DE)**<br>• **Raeven, Leon Jean Renier Maria<br>2642 AV Pijnacker (NL)**<br>• **Sprenger, Georg<br>52428 Jülich (DE)**<br>• **Takors, Ralf<br>52399 Merzenich (DE)**<br><br>(74) Representative: **Jacobs, Monique S.N.<br>DSM Patents & Trademarks<br>Office Geleen<br>Postbus 9<br>6160 MA Geleen (NL)** |

(54) **Process for the production of an aromatic amino acid metabolite or derivative thereof**

(57)    The invention relates to a process for the production of an aromatic amino acid metabolite or derivative thereof by aerobic fermentation of <u>Escherichia coli,</u> which fermentation comprises a growth and a production phase and in which fermentation glucose and L-tyrosine are controlled, wherein during at least part of the production phase, the glucose concentration in the fermentation medium is controlled within the range of 1-20 g/l and the L-tyrosine concentration in the fermentation medium is controlled below 36 mg/l.

EP 1 318 199 A1

Printed by Jouve, 75001 PARIS (FR)

**Description**

The invention relates to a process for the production of an aromatic amino acid metabolite or derivative thereof by aerobic fermentation of Escherichia coli, which fermentation comprises a growth and a production phase and in which fermentation glucose and L-tyrosine are controlled.

For purpose of this application, the term "an aromatic amino acid metabolite or derivative thereof" means, any metabolite, which is an intermediate in or an end product of the aromatic amino acid pathway or a product derived from such a metabolite, with the exception of L-tyrosine and products derived from L-tyrosine. Examples of aromatic amino acid metabolites or derivatives thereof are, for example, 3-deoxy-D-arabino-heptulosonate-7-phosphate, 3-dehydroquinate, quinic acid, hydroquinone, 3-dehydroshikimate, catechol, adipic acid, a cyclitol, shikimate, shikimate-3-phosphate, 5-enolpyruvate-3-phosphate, chorismate, L-tryptophan, indigo, prephenate, L-p-hydroxyphenylglycine, L-phenylglycine, D-p-hydroxyphenylglycine, D-phenylglycine, phenylpyruvate, L-phenylalanine, D-phenylalanine, anthranilate, phosphoribosyl anthranilate, 1-(o-carboxyphenylamino)-1-deoxyribulose-5-phosphate, indole 3-glycerol phosphate, indole, 4-hydroxyphenylpyruvate. Preferably, the aromatic amino acid metabolite or derivative thereof is L-phenylalanine, D-phenylalanine, D-hydroxyphenylglycine, D-phenylglycine or shikimate. More preferably, the aromatic amino acid metabolite or derivative thereof is L-phenylalanine.

The term "aerobic fermentation" means, that oxygen is present and not limiting during the whole fermentation.

The growth phase in the Escherichia coli fermentation is the phase in which the biomass concentration of the Escherichia coli fermentation medium increases. The biomass concentration can be determined by measurement of the optical density of the fermentation medium at 620 nm ($OD_{620}$). The production phase in the Escherichia coli fermentation is the phase in which the product, the aromatic amino acid metabolite or derivative thereof, is produced. The growth and production phase can occur one after the other, but in practice the growth and production phase overlap.

The term "fermentation medium" means the liquid fermentation medium with all its components, including Escherichia coli cells.

A process for the production of an aromatic amino acid metabolite or derivative thereof by aerobic fermentation of Escherichia coli, which fermentation comprises a growth and a production phase and in which fermentation glucose and L-tyrosine are controlled is known from Takagi et al., (1996) Biotechnology and Bioengineering Vol. 52, p 653-660. Said article describes an aerobic fermentation process for the production of L-phenylalanine by a recombinant Escherichia coli AT2471, in which fermentation the glucose concentration in the fermentation medium was controlled below 0.1 g/l after depletion of the initial amount of glucose, 10 hours after the start of the fermentation (coincides approximately with the start of the production phase) and the L-tyrosine feed was controlled at 100 mg of a solution of 2 g/l L-tyrosine per hour (corresponding to the addition of approximately 0.2 g L-tyrosine per hour) to a volume of 13.5 l fermentation medium after depletion of the initial L-tyrosine, which was after 30 hours (coincides approximately with the end of the growth phase).

In the experiments of Takagi et al., (1996), glucose was controlled by keeping the glucose concentration in the fermentation medium below 0.1 g/l, because it was believed that for high productivity, it is essential to prevent the accumulation of acetic acid. The general believe that the glucose concentration needs to be controlled at a low level in order to prevent acetate accumulation is also conformed by others (among others by Sakamoto et al., (1994) J. Ferm. Bioeng. Vol. 78, p 304-309, Shiloach et al., (1996) Biotechnol. Bioeng. Vol 49, p 421-428, Luli et al., (1990), Appl. Environ. Microbiol. Vol 56, p 1004-1011).

Accumulation of acetic acid caused by the excretion of acetate by Escherichia coli is unwanted as, in the fermentation of an Escherichia coli strain for the production of an aromatic amino acid metabolite or derivative thereof, it leads among others to a decreased growth rate, a decreased final cell concentration [Kleman et al., 1991, Appl. Environ. Microbiol. 57(4) 918-923] and a decreased uptake of glucose [Xu B., et al., 1999 Biotechnol. Prog. 15, 81-90] and thereby to a decrease in total yield of the process (product/substrate in molar %).

It is thus known that acetate may inhibit fermentation. For purpose of the invention, the extracellular acetate concentration in the fermentation medium at which acetate interferes with the fermentation process is called inhibiting acetate concentration. The inhibiting acetate concentration is strain dependent and is for purpose of this invention defined as the concentration at which the maximal production rate of the organism is halved. The person skilled in the art is aware that other definitions for the inhibiting acetate concentration also exist. For example, Xu et al., 1999. Biotechn. Prog. Vol. 15, p 81-90 defined the inhibiting acetate concentration as the concentration at which the maximal cell growth is halved and determined an inhibiting acetate concentration ($k_i$) of 9 g/l for the Escherichia coli K12 derived strain W3110. In a preferred ambodiment, the fermentation is performed until the inhibiting acetate concentration is reached; the formed product can then be isolated according to methods known to the person skilled in the art.

L-Tyrosine is generally known to be responsible for the feed-back regulation of the aromatic amino acid pathway. Such feed-back regulation is two-fold: (1) it inhibits some enzymes in the aromatic amino acid pathway, which are feed-back regulated by L-tyrosine (for example 3-desoxy-D-arabino-heptusonate-7-phosphate synthase, also known as DAHP synthase) and (2) it has an activating effect on the tyrR regulon, which under the influence of L-tyrosine produces a protein, which represses the expression of some of the genes expressing the enzymes necessary in the aromatic amino acid pathway. It has been found by Förberg *et al.,* (1988) J. Biotechnol. Vol 8, p 291-300 that an L-tyrosine concentration of 36 mg/l causes 86% inhibition of DAHP synthase and that at L-tyrosine concentrations as low as 1.8 mg/l enzyme synthesis is repressed to 44%.

The presence of too much L-tyrosine therefore also strongly affects the production of an aromatic amino acid metabolite or derivative thereof. To limit the amount of L-tyrosine present during the production of an aromatic amino acid metabolite or derivative by aerobic fermentation of Escherichia coli, usually an Escherichia coli strain is used, which is auxotrophic for L-tyrosine (i.e. the strain does not produce any L-tyrosine by itself). Takagi *et al.* (1996) kept the L-tyrosine feed at a constant value (at approximately 0.2 g/h) after depletion of the initial L-tyrosine (approximately after 30 hours of fermentation) as L-tyrosine is needed for cell maintenance, but too much L-tyrosine leads to a decrease in production of L-phenylalanine.

A drawback of the control of glucose in the fermentation medium below 0.1 g/l is that in large-scale fermentations the maximal production rate can never be achieved throughout the whole fermentation medium if glucose is the main carbon source used by the microorganism for the production of an aromatic amino acid metabolite or derivative thereof. This is caused by the fact that in large-scale fermentations the distribution of glucose (and other nutrients) is never homogeneous throughout the fermentation medium. Therefore, control of glucose below 0.1 g/l in a large scale fermentation leads to the existence of local zones in the fermentation medium where the glucose concentration is far from sufficient to ensure a maximal production rate of the product produced by a micro organism using glucose as the main carbon source. If the glucose concentration is not sufficient to ensure a maximal production rate, the glucose concentration is called limiting. If the glucose concentration is limiting, there will also be a decrease in selectivity of the microorganism towards its product, meaning that there will be more byproduct formation. With byproduct is meant everything else that is formed from glucose with the exception of the product itself (an aromatic amino acid metabolite or derivative thereof).

It is an object of the invention to provide a process for the production of an aromatic amino acid metabolite or derivative thereof by aerobic fermentation of Escherichia coli, which fermentation comprises a growth and a production phase and in which fermentation glucose and L-tyrosine are controlled in which the glucose concentration is not limiting anywhere in the fermentation medium, in which inhibition by acetate is prevented and in which the inhibiting effect of L-tyrosine is limited.

The object of the invention is achieved by control of the glucose concentration in the fermentation medium within the range of 1-20 g/l and by control of the L-tyrosine concentration in the fermentation medium below 36 mg/l, during at least part of the production phase.

Surprisingly, it has been found that even when the glucose concentration in the fermentation medium is controlled at a value above 1 g/l, the acetate concentration does not become inhibiting for at least 10 hours in the production phase.

The glucose concentration in the fermentation medium is controlled according to the invention within the range of 1-20g/l, preferably within the range of 1-15 gl/, more preferably within the range of 3-10 g/l, most preferably within the range of 4-6 g/l. Preferably, the variations in the glucose concentration vary within a narrower range (subrange) falling within a glucose concentration range of 1-20 g/l. Preferably, the upper and lower limits of the subrange are not more than 10 g/l apart, this means for example that the glucose concentration is controlled between 3-13 g/l or between 7-17 g/l or between 1-11 g/l. More preferably, the upper and lower limits of the subrange are not more than 5 g/l apart, this means for example a glucose concentration between 3-8 g/l, between 7-12 g/l, between 1-6 g/l. Even more preferably, the upper and lower limits of the subrange are not more than 2 g/l apart, this means for example a glucose concentration variation between 3-5 g/l, between 16-18 g/l, between 4-6 g/l. Most preferably, the upper and lower limits of the subrange are not more than 1 g/l apart, this means for example a glucose concentration variation between 5-6 g/l, between 17-18 g/l, between 1-2 g/l. Best results are obtained for subranges falling within the range of 3-10 g/l, specifically 4-6 g/l.

The glucose concentration in the fermentation medium is preferably controlled after the initial glucose has reached a value within the chosen control range. The initial glucose concentration in the fermentation medium is preferably chosen from the range of 10-40 g/l, more preferably from the range of 15-35 g/l. In a preferred embodiment of the invention glucose is controlled during the entire production phase.

L-tyrosine control is preferably started after the initial L-tyrosine concentration is at or below the chosen upper L-tyrosine concentration limit and preferably started before the initial amount of L-tyrosine is fully depleted. The initial L-tyrosine concentration is preferably chosen within the range of 100-380 mg/l, more preferably within the range of 200-300 mg/l. The timing of the start of the L-tyrosine control is not critical, but can be after 3 hours of fermentation, preferably after 4 hours of fermentation, more preferably after 5 hours of fermentation, most preferably after 6 hours of fermentation. Surprisingly, it has been found that if L-tyrosine control is started much earlier in the fermentation than at 30 hours as described by Takagi *et al.* (1996), the product/substrate yield is increased. L-tyrosine is preferably controlled at a L-tyrosine concentration in the fermentation medium below 36 mg/l fermentation medium, more preferably below 20 mg/l, even more preferably below 10 mg/l.

In a preferred embodiment of the invention, L-tyrosine control is preferably carried out as long as the fermentation is in the growth phase. When the fermentation is no longer in the growth phase (typically after 20 hours of fermentation), a constant L-tyrosine feed is optionally started. Preferably the constant amount of L-tyrosine fed into a bioreactor containing the fermentation medium with 1 g/l cell dry weight concentration (CDW) is chosen within the range of 0.01-5 $g_{tyrosine}$ per hour. Accordingly, if a bioreactor containing 10 I fermentation medium has a CDW of 30 g/l (total CDW of 300g), the amount of L-tyrosine fed per hour is preferably chosen within the range of 0.003-1.5 kg. CDW can be determined as described in materials and methods.

Escherichia coli strains suitable for use in the process according to the invention are all Escherichia coli strains, which have the ability to convert glucose into an aromatic amino acid metabolite or derivative thereof and that are L-tyrosine auxotrophic. For the production of an aromatic amino acid metabolite or derivative thereof, it is preferable that the strain has an impeded downstream pathway as from the desired endproduct, which downstream pathway (e. g. leading to shikimate-3-phosphate and further in the case of shikimate production) would be leading to the further conversion of the desired end product (e.g. shikimate). Alternatively, the desired endproduct is isolated from the producing cells. More preferably, in addition to the impediment of a downstream pathway, pathways leading to other products than the desired endproduct (branching pathway) are also impeded (e.g. the pathway to L-tyrosine in the case of L-phenylalanine as the desired endproduct). All of the above measures are aimed at achieving a high efficiency of flux of the glucose into the desired end product (an aromatic amino acid metabolite or derivative thereof). It is clear to the person skilled in the art that there may be other ways than the above described ways, to achieve similar results.

Examples of suitable Escherichia coli strains are for example L-phenylalanine producing strains, which are based on Escherichia coli K12 strains, preferably Escherichia coli W3110, more preferably Escherichia coli LJ110 (Zeppenfeld *et al.* (2000), J. Bacteriol. Vol 182, p 4443-4452. Other examples of Escherichia coli strains capable of producing for example L-tryptophane, 3-dehydroshikimic acid, shikimic acid and D-phenylalanine are described in Bongaerts *et al.* (2001) Metabolic Engineering (2001) vol. 3, p 289-300.

An example of an Escherichia coli strain, which has the ability to produce a product from the aromatic amino acid pathway, more specifically shikimate 3-phosphate from glucose and in which the downstream pathway leading to the further conversion of shikimate 3-phosphate into 5-enolpyruvyl-shikimate-3-phosphate is impeded is the *E. coli* strain AB2829 (the CGSC-strain, Pittard *et al.* (1966) J Bacteriol. Vol 92, p 1494-1508). This strain has a deletion in the gene (*aro*A) encoding the 5-enolpyruvyl-shikimate-3-phosphate synthase (EPSP-synthase) responsible for the conversion of shikimate 3-phosphate into 5-enolpyruvyl-shikimate-3-phosphate.

Further examples of Escherichia coli strains with the ability to produce L-phenylalanine from glucose and in which the branching pathway leading to a different product has been impeded are Escherichia coli K12 strains 4pF26 and 4pF69, which have a deletion in the gene (*tyr*A) encoding chorismate mutase/prephenate dehydrogenase, which under normal circumstances causes the conversion of prephenate into 4-hydroxyphenylpyruvate (a precursor for the production of L-tyrosine).

To limit the inhibiting effect of L-tyrosine, mostly the wild type (WT) gene *aro*F$^{WT}$ (encoding an L-tyrosine feed-back regulated 3-desoxy-D-arabino-heptulosonate-7-phosphate synthase) is deleted from the genome of Escherichia coli and complemented in the Escherichia coli strain, on for example a vector, or by insertion into the genome etc., with the L-tyrosine feed-back resistant (FBR) variant of the gene *aro*F$^{FBR}$.

Surprisingly, it has been found that in the present invention, use of an Escherichia coli strain with wild type *aro*F-gene (*aro*F$^{WT}$) leads to a higher product/glucose yield (in molar %) of L-phenylalanine than the use of an Escherichia coli strain with a deleted *aro*F$^{WT}$-gene complemented with the *aro*F$^{FBR}$. Therefore, in a preferred embodiment of the invention, an Escherichia coli strain, in which *aro*F$^{WT}$ is expressed, for example on a vector or in the Escherichia coli genome, is used.

The reaction conditions at which the process according to the invention is carried out are reaction conditions normally chosen for aerobic fermentation of Escherichia coli and are known to the person skilled in the art, with temperatures chosen within the range of 10 - 70 °C, preferably within the range of 25-40°C, most preferably within the range of 33 - 37°C and with pH ranges from 5-9, preferably from 6-8, most preferably from 6.6-6.8. Medium compositions are also known to the person skilled in the art; a very suitable medium is the M9 medium (Sambrook *et al.* (1989) Molecular Cloning: A Laboratory Manual. 2nd, ed., Cold Spring Harbor Laboratory, Cold Spring Harbor Laboratory Press, Cold Spring Harbor, NY) is used.

The glucose concentration can suitably be monitored directly with, for example, the method as described in materials and methods and the glucose feed can be adjusted accordingly.

The L-tyrosine concentration can suitably be monitored indirectly by measurement of measurable variables with a linear or non-linear correlation (e.g. exhaust gas signals, for instance $CO_2$ emission rate or the oxygen uptake rate) with the L-tyrosine concentration. The correlation can be empirically established and can then be used to adjust the L-tyrosine feed such that the L-tyrosine concentration remains below the chosen set-point and such that the yield of the aromatic amino acid metabolite or derivative thereof is optimized.

In a special embodiment of the invention, the L-tyrosine concentration is adjusted according to the following linear equation (1):

$$
\dot{V}_{tyr}\left[\frac{g}{Lh}\right]=\frac{A\left[\dfrac{mmol}{Lh}\right]-k\left[\dfrac{mmol}{Lh}\right]}{m\left[\dfrac{mmol}{g}\right]} \tag{1}
$$

The feed of L-tyrosine ($V_{tyr}$) can be adjusted according to A (which is the measured oxygen uptake rate (OUR) or, alternatively, the $CO_2$ emission rate (CER)) to keep the L-tyrosine concentration below the chosen control concentration. In equation 1, *m* and *k* represent controlling parameters that could be adjusted, for instance, to increase L-tyrosine limitation by increasing *m* or *k* values. The controlling parameters should best be chosen such that the yield of the aromatic amino acid metabolite or derivative thereof is optimized. m values are typically chosen within the range of 0.1 - 4; *k* values are typically chosen within the range of 20-40. The optimal *m* and the *k* values can be empirically determined. For a good control of the L-tyrosine concentration by using this method, other state variables like pH, temperature or dissolved oxygen concentration (DO) should be kept constant.

The invention is illustrated by way of the following examples. However, these examples are not meant to restrict the invention.

Examples

Materials and methods

Biological systems

Based on *E. coli* K 12 LJ110 (Zeppenfeld et al. 2000), two production strains with the synonyms *E. coli* aroF-fbr (coding for the genotype Δ(*pheA tyrA aroF*)/pJF119EH *aroF^{fbr} pheA^{fbr} aroL^{wt}*) and *E. coli* aroF-wt (coding for the genotype Δ(*pheA tyrA aroF*)/pJF119EH *aroF^{wt} pheA^{fbr} aroL^{wt}*) were constructed, using the plasmid pJF119EH (Fürste et al., 1986). In both strains, the genes *pheA* (encoding chorismate mutase/ prephenate dehydratase), *tyrA* (encoding chorismate mutase/prephenate dehydrogenase) and *aroF* (encoding DAHP-synthase (3-desoxy-D-arabino-heptusonate-7-phosphate)) were deleted in chromosome. In *E. coli* three isoenzymes AroF (feedback inhibited by tyrosine), AroG (feedback inhibited by phenylalanine) and AroH (feedback inhibited by tryptophane) enable DAHP-synthase activity. In *E. coli* aroF-fbr a tyrosine resistant derivate (*aroF^{fbr}*) of DAHP-synthase was inserted on the plasmid. The plasmid of *E. coli* aroF-wt contained the tyrosine sensitive *aroF^{wt}* instead of *aroF^{fbr}*. Additionally phenylalanine feed-back-resistant *pheA^{fbr}* and native *aroL^{wt}* (encoding shikimate kinase II) were inserted on pJF119EH to avoid PheA feedback inhibition by phenylalanine and to increase AroL activity. Due to Δ*tyrA* the production strains are tyrosine auxotrophic. Using the expression vector pJF119EH, the strains possess ampicillin resistance and are IPTG-inducible (glucose resistant tac-promotor is used).

Media composition

Fermentation medium: 3.0 g/l $MgSO_4$ x 7 $H_2O$, 0.015 g/l $CaCl_2$ x $H_2O$, 3.0 g/l $KH_2PO_4$, 1.0 g/l NaCl, 5.0 g/l $(NH_4)_2SO_4$, 0.075/0.1 g/l $FeSO_4$ x 7 $H_2O$/Na-citrate, 0.075 g/l thiamine, 0.3 g/l L-tyrosine, 0.1 g/l ampicilline, 15 g/l glucose and 1.5 ml/l trace element solution containing 2.0 g/l $Al_2(SO_4)_3$ x 18 $H_2O$, 0.75 g/l $CoSO_4$ x 7 $H_2O$, 2.5 g/l $CuSO_4$ x 5 $H_2O$, 0.5 g/l $H_3BO_3$, 24 g/l $MnSO_4$ x $H_2O$, 3.0 g/l $Na_2MoO_4$ x 2 $H_2O$, 2.5 g/l $NiSO_4$ x 6 $H_2O$ and 15.0 g/l $ZnSO_4$ x 7 $H_2O$.
Precultivation medium: The same medium was used as for fermentation except for the following changes: 0.3 g/l $MgSO_4$ x 7 $H_2O$, 0.1 g/l NaCl, 0.0075 g/l thiamine x HCl, 0.08 g/l L-tyrosine, 5.0 g/l glucose and additionally 12 g/l $K_2HPO_4$ (final pH 7.2).

Precultivation

The cyroculture was stored at -80°C in Luria-Bertani (LB) medium containing 50% glycerol. 250 ml precultivation medium was filled into 1000 ml shake flasks, 1.0 ml from feedstock was inoculated and cultivated for 16 h at 37°C on a shaking flask incubator (145 rpm).

Cultivation

Glucose was used as the sole carbon source in the defined medium. pH was controlled by 25 % ammonia water titration. Glucose and L-tyrosine (due to the L-tyrosine auxotrophy of the strain) were added to the bioreactor to ensure cell growth during batch phase. Two separate feeds for L-Tyr (25 g/L L-tyrosine feed, dissolved in 5% ammonia water) and glucose (700 g/L) were then started to extend growth phase. The feed rates of both substrates were automatically adapted by control strategies, implemented in the process control system. When the feeding phase was started, L-Phe production was induced by addition of IPTG (final concentration 100 μM). Cell growth was stopped by L-Tyr limitation (approximately at $OD_{620} \approx 80$) ensuring an ongoing tyrosine supply for cell maintenance with a feed rate of 150 mg/h until the end of fermentation.

Cultivations were conducted in a 20.0 L bioreactor (ISF 200, Infors; Switzerland) with 10% inoculation; 7.5 L initial volume, cultivation temperature 37°C and pH 6.5. After sterilisation of the bioreactor and peripheral equipment and calibration of pH-, DO-sensors and exhaust gas analyser, 6.75 L of fermentation medium was directly filtrated into the bioreactor via a dead-end microfiltration unit (0.2 μm cut off, Satorbran, Satorius, Germany).

Off-line analyses

Off-line analyses were performed from samples taken at 1.5 to 2.5 h intervals. Cell concentration was measured by a photospectrometer (Shimadzu UV-160, Germany) at 620 nm after appropriate dilution. Cell dry weight (CDW) was measured by filtration of 2.5 to 10.0 ml fermentation medium through a preweighted microfilter (0.2 μm cut off, Schleicher&Schuell; Germany). After drying the filter for 24 h at 80°C and postweighing, the cell dry mass was calculated. Immediately after sampling, glucose was measured by an enzyme-based biosensor appliance Accutrend® (Hoffmann LaRoche Diagnostics; Switzerland) after appropriate dilution. Acetic acid concentration was measured by HPLC (Sycam; Germany) using an ion-exclusion column (Aminex-HPX-87H, BioRad; Germany) and a photospectrometric detector at 215 nm (S3300, Sycam; Germany). Amino acids concentrations (L-Phe and L-Tyr) were measured by prederivatisation with the amino-specific reactant orthophthal-dialdehyde (OPA) and mercapto-ethanol followed by HPLC (Sycam; Germany) using a reversed phase column (Lichrospher 100 RP 18-5 EC, Merck; Germany) and a fluorescence detector (RF-535, Shimadzu; Germany). Because L-Phe and L-Tyr concentrations differ from each other by order of magnitudes, different dilutions of one sample were necessary.

On-line glucose measurement

Three peristaltic pumps (U 501 and U 101, Watson&Marlow; Germany) were used to ensure continuous sampling. With a flow rate of 800 mL/min fermentation medium was pumped through a by-pass (total volume: ≈ 20 mL, mean residence time: ≈ 2s) containing a cross-flow hollow fibre ultrafiltration unit (500 kDa cut-off, 23 cm² filtration area, Schleicher&Schuell, Germany). 1.0 - 2.0 mL/min cell-free permeate was drained off and pumped to the manifold of the sequential on-line glucose measurement system (OLGA, IBA GmbH; Germany) where 10 μL samples were drained off for analysis every 120s. Unused permeate was recycled into the bioreactor. Hence, not more then 100 mL permeate was used for on-line glucose measurement during fermentation. The whole sampling system was sterilised with 1 M NaOH at 50°C for 30 min.

Process control

Control of standard process parameters was performed by Infors (Switzerland) devices. Main data acquisition was realised by LabView (National Instruments; U.S.A.) that was combined with MEDUSA (IBT software) and the OLGA control system. Signals of on-line glucose measurement were sent from OLGA via LabView to MEDUSA where a control system consisting of Kalman-filter and minimal variance controller (Bastin et al., 1984) estimated optimal glucose feeding rates to meet the predefined glucose setpoint. Glucose feeding rate was automatically adjusted with aid of a feeding system (Satorius; Germany). Tyrosine was indirectly controlled during growth phase using an on-line estimation of the volume specific oxygen uptake rate (*OUR*) by measurement of $O_2$-/$CO_2$ in exhaust gas (Binos 100 2M, Leybold, Germany), bioreactor weight and air flow rate. A volume specific L-Tyr consumption rate was estimated in MEDUSA and a feed containing 25 g/L was used for its adjustment with aid of a feeding system (Satorius; Germany).

Example I

The fermentation was performed according to what is described in materials and methods. Glucose control was started when the initial glucose concentration was decreased to the chosen glucose control value (0.1; 5.0; 15.0; 30.0) at approximately 10 hours from the start of the fermentation. Tyrosine control via on-line measurement of the OUR and according adjustment of the L-tyrosine feed was started at 6 hours from the start of the fermentation to keep the L-tyrosine concentration in the fermentation medium below 20mg/l. 100μM IPTG was added after achieving an optical density at 620 nm ($OD_{620}$) of 10-15 to induce L-phenylalanine production (at approximately 6 hours after the start of the fermentation). After the L-tyrosine control via on-line measurement of the OUR and according adjustment of the L-tyrosine feed was stopped, a continuous feed of 100mg/h of the L-tyrosine solution, containing 25 g/l L-L-tyrosine, dissolved in 5% ammonia water, was started. The L-phenylalanine (L-Phe) concentration and acetate (Ac) concentration in the fermentation medium in different points in time as a result of the control of glucose concentration in the fermentation medium at 0.1, 5, 15 and 30 g/l and control of L-tyrosine below 36 mg/l are shown in Table 1.

Table 1

| Time (hour) | glucose concentration in the fermentation medium (g/l) | | | | | | | |
|---|---|---|---|---|---|---|---|---|
| | 0.1 | | 5 | | 15 | | 30 | |
| | L-Phe (g/l) | Ac (g/l) | L-Phe (g/l) | Ac (g/l) | L-Phe (g/l) | Ac (g/l) | L-Phe (g/l) | Ac (g/l) |
| 0.5 | 0.0 | 0.2 | 0.0 | 0.1 | 0.0 | 0.1 | 0.0 | 0.1 |
| 10 | 1.6 | 0.6 | 2.8 | 1.0 | 2.3 | 0.0 | 2.5 | 1.6 |
| 27 | 16.0 | 0.0 | 25.2 | 0.8 | 22.4 | 1.3 | 16.7 | 8.0 |
| 35 | 24.7 | 0.0 | 31.9 | 1.0 | 30.5 | 5.5 | 16.6 | 24.4 |
| 50 | 31.6 | 1.3 | 34.4 | 1.9 | 31.2 | 24.9 | 16.1 | 40.9 |

Example II

The fermentation was performed according to what is described in materials and methods with the 4pF69 strain (with wild type *aro*F) and with the 4pF26 strain (with feed-back resistant *aro*F). Glucose control was started when the initial glucose concentration was decreased to a glucose concentration of 5 g/l fermentation medium at approximately 10 hours from the start of the fermentation. L-tyrosine control via on-line measurement of the OUR and according adjustment of the L-tyrosine feed was started at 6 hours from the start of the fermentation to keep the L-tyrosine concentration in the fermentation medium below 20mg/l. 2 different m-values were chosen (m = 1.0, m = 1.5), the k-value was set at 30. 100μM IPTG was added after achieving an optical density at 620 nm ($OD_{620}$) (at approximately 6 hours after the start of the fermentation) to induce L-phenylalanine production. After the L-tyrosine control via on-line measurement of the OUR and according adjustment of the L-tyrosine feed was stopped, a continuous feed of 100mg/h of the L-tyrosine solution, containing 25 g/l L-tyrosine, dissolved in 5% ammonia water, was started. The L-phenylalanine (L-Phe) concentration in the fermentation medium in different points in time as a result for the different m-values and for the different strains are shown in Table 2.

Table 2

| Time (hour) | Strain type | | | |
|---|---|---|---|---|
| | 4pF69 (*aro*F wild | type) | 4pF26 (aroF feed-back resistant) | |
| | m = 1.5 | m = 1.0 | m = 1.5 | m = 1.0 |
| | yield product/substrate (mol/mol%) | | | |
| 36 | 20 | 19 | 13 | 18 |
| 48 | 17 | 16 | 11 | 15 |

**Claims**

1.  Process for the production of an aromatic amino acid metabolite or derivative thereof by aerobic fermentation of Escherichia coli, which fermentation comprises a growth and a production phase and in which fermentation glucose and L-tyrosine are controlled, **characterized in that** during at least part of the production phase, the glucose concentration in the fermentation medium is controlled within the range of 1-20 g/l and **in that** the L-tyrosine concentration in the fermentation medium is controlled below 36 mg/l.

2.  Process according to claim 1, **characterized in that** the glucose concentration is controlled within the range of 3-10 g/l

3. Process according to claim 1 or 2, **characterized in that** within the glucose concentration range of 1-20 g/l or of 3-10 g/l, the glucose concentration is controlled in a subrange with its upper and lower limits not more than 5 g/l apart.

4. Process according to any of claims 1-2, **characterized in that** the glucose concentration is controlled within the range of 4-6 g/l.

5. Process according to any of claims 1-2, or 4, **characterized in that** the L-tyrosine concentration is controlled below 20 mg/l.

6. Process according to any of claims 1, 2, 4 or 5, **characterized in that** the fermentation is performed until the concentration of acetate, which is produced as a byproduct of the fermentation, reaches the inhibiting acetate concentration.

7. Process according to any of claims 1, 2, 4-6, **characterized in that** glucose control is performed during the entire production phase.

8. Process according to any of claims 1, 2, 4-7, **characterized in that** the tyrosine concentration in the fermentation medium is controlled as long as the fermentation is in the growth phase.

9. Process according to any of claims 1, 2, 4-8, **characterized in that** after the tyrosine concentration control is stopped, a continuous tyrosine feed is started.

10. Process according to any of claims 1, 2, 4-9, **characterized in that** the continuous tyrosine feed is chosen such that the amount of L-tyrosine fed is between 0.01-5 g per hour per cell dry weight concentration.

11. Process according to any of claims 1, 2, 4-10, **characterized in that** the tyrosine concentration in the medium is controlled by use of an empirically established correlation between a measurable variable to adjust the tyrosine feed and consequently the tyrosine concentration in the fermentation medium.

12. Process according to any of claims 1, 2, 4-11, **characterized in that** the tyrosine concentration in the medium is controlled by adjustment of the tyrosine feed according to the following equation:

$$\dot{V}_{tyr}\left[\frac{g}{Lh}\right] = \frac{A\left[\frac{mmol}{Lh}\right] - k\left[\frac{mmol}{Lh}\right]}{m\left[\frac{mmol}{g}\right]} \qquad (1)$$

wherein A represents the oxygen uptake rate in the fermentation (OUR) or the $CO_2$ emission rate (CER), $V_{tyr}$ represents the tyrosine feed and wherein $k$ and m represent controlling parameters.

13. Process according to any of claims 1, 2, 4-12, **characterized in that** Escherichia coli W3110 is used.

14. Process according to any of claims 1, 2, 4-13, **characterized in that** an aromatic amino acid metabolite or derivative thereof is L-phenylalanine.

15. Process according to 14, **characterized in that** in Escherichia coli, $aro$F$^{WT}$ is expressed.

16. Method for control of the tyrosine concentration by use of an empirically established correlation between a measurable variable to adjust the tyrosine feed and consequently the tyrosine concentration in the fermentation medium.

17. Method according to claim 16, **characterized in that** the tyrosine feed is adjusted according to the following equation:

$$\dot{V}_{tyr}\left[\frac{g}{Lh}\right] = \frac{A\left[\dfrac{mmol}{Lh}\right] - k\left[\dfrac{mmol}{Lh}\right]}{m\left[\dfrac{mmol}{g}\right]} \qquad (1)$$

wherein A represents the oxygen uptake rate in the fermentation (OUR) or the $CO_2$ emission rate (CER), $V_{tyr}$ represents the tyrosine feed and wherein $k$ and m represent controlling parameters.

## EUROPEAN SEARCH REPORT

**European Patent Office**

**Application Number**

EP 01 20 4720

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X | TAKAGI MUTSUMI ET AL: "Control of L-phenylalanine production by dual feeding of glucose and L-tyrosine" BIOTECHNOL BIOENG;BIOTECHNOLOGY AND BIOENGINEERING DEC 20 1996 JOHN WILEY & SONS INC, NEW YORK, NY, USA, vol. 52, no. 6, 20 December 1996 (1996-12-20), pages 653-660, XP002195861 | 1-10,14 | C12P13/22 C12M1/36 C12Q3/00 |
| Y | * the whole document * | 11,12, 16,17 | |
| X | FORBERG C ET AL: "CORRELATION OF THEORETICAL AND EXPERIMENTAL YIELDS OF PHENYLALANINE FROM NON-GROWING CELLS OF A REC ESCHERICHIA-COLI STRAIN" JOURNAL OF BIOTECHNOLOGY, vol. 7, no. 4, 1988, pages 319-331, XP002195860 ISSN: 0168-1656 * abstract * * page 323; figure 1 * | 1,2,4,5, 7,8, 13-15 | |
| Y | DATABASE BIOSIS 'Online! BIOSCIENCES INFORMATION SERVICE, PHILADELPHIA, PA, US; 1984 HONG K T ET AL: "CONTROL OF SUGAR FEEDING FOR GLUTAMIC-ACID FERMENTATION" Database accession no. PREV198478034818 XP002195924 * abstract * & JOURNAL OF FERMENTATION TECHNOLOGY, vol. 62, no. 1, 1984, pages 49-54, ISSN: 0385-6380 | 11,12, 16,17 | |

**TECHNICAL FIELDS SEARCHED (Int.Cl.7)**

C12P
C12M
C12Q

-/--

The present search report has been drawn up for all claims

| Place of search | Date of completion of the search | Examiner |
|---|---|---|
| THE HAGUE | 21 May 2002 | Blanco Urgoiti, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 01 20 4720

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | KONSTANTINOV KONSTANTIN B ET AL: "Physiologically motivated strategies for control of the fed-batch cultivation of recombinant Escherichia coli for phenylalanine production" J FERMENT BIOENG;JOURNAL OF FERMENTATION AND BIOENGINEERING 1991, vol. 71, no. 5, 1991, pages 350-355, XP001064898 * the whole document * | | |
| D,A | BONGAERTS J, ET AL: " Metabolic engineering for microbial production of aromatic amino acids. " METABOLIC ENGINEERING, vol. 3, October 2001 (2001-10), pages 289-300, XP002195863 | | |
| D,A | ZEPPENFELD TIM ET AL: "Glucose transporter mutants of Escherichia coli K-12 with changes in substrate recognition of IICBGlc and induction behavior of the ptsG gene." JOURNAL OF BACTERIOLOGY, vol. 182, no. 16, August 2000 (2000-08), pages 4443-4452, XP002195864 ISSN: 0021-9193 | | TECHNICAL FIELDS SEARCHED (Int.Cl.7) |

The present search report has been drawn up for all claims

| Place of search | Date of completion of the search | Examiner |
|---|---|---|
| THE HAGUE | 21 May 2002 | Blanco Urgoiti, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
......................................................................
& : member of the same patent family, corresponding document

EPO FORM 1503 03.82 (P04C01)